# EUROPÄISCHE PATENTSCHRIFT

(11) **EP 3 133 054 B1**
(45) Veröffentlichungstag und Bekanntmachung des Hinweises auf die Patenterteilung: **07.03.2018**
(21) Anmeldenummer: 16182752.2
(22) Anmeldetag: 04.08.2016
(51) Int. Cl.: C07C 41/30, C07C 213/08

(54) **VERFAHREN ZUR HERSTELLUNG VON SYMMETRISCHEN PINCERLIGANDEN AUS DER GRUPPE DER M-TERPHENYLVERBINDUNGEN**
METHOD FOR THE PREPARATION OF SYMMETRICAL PINCER LIGANDS FROM THE GROUP OF M TERPHENYL COMPOUNDS
PROCEDE DE FABRICATION DE LIGANDS PINCES SYMETRIQUES PROVENANT DU GROUPE DES COMPOSES DE TERPHENYLE M

(30) Priorität: 21.08.2015 DE 102015216000
(43) Veröffentlichungstag der Anmeldung: 22.02.2017
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Waldvogel, Siegfried R., 55435 Gau-Algesheim (DE); Elsler, Bernd, 53127 Bonn (DE); Wiebe, Anton, 56567 Neuwied (DE)

(56) Entgegenhaltungen:
- AXEL KIRSTE ET AL: "Efficient Anodic and Direct Phenol-Arene C,C Cross-Coupling: The Benign Role of Water or Methanol", JOURNAL OF THE AMERICAN CHEMICAL SOCIETY, Bd. 134, Nr. 7, 22. Februar 2012 (2012-02-22), Seiten 3571-3576, XP055114642, ISSN: 0002-7863, DOI: 10.1021/ja211005g

## Beschreibung

Die vorliegende Erfindung betrifft ein Verfahren zur Herstellung von Verbindungen der Formel ABA 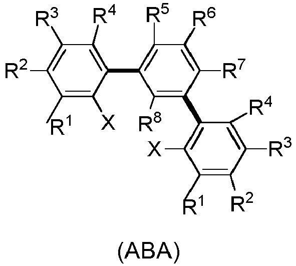 durch Umsetzen einer Verbindung der Formel (A) 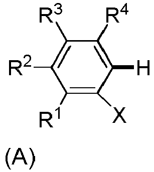 mit einer Verbindung der Formel (B) 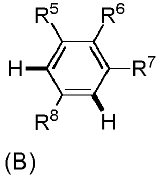 mit X = -OH oder -NR'R" und R' = H oder eine Schutzgruppenfunktion und R" = H oder eine Schutzgruppenfunktion und R' und R" miteinander kovalent verknüpft sein können und R¹, R², R³, R⁴, R⁵, R⁶, R⁷ und R⁸ unabhängig voneinander ausgewählt sind aus der Gruppe enthaltend Wasserstoff, Kohlenwasserstoffreste und Heteroatome aufweisende Kohlenwasserstoffreste, wobei benachbarte Reste aus der Gruppe der Reste R¹ bis R⁸ über eine kovalente Bindung verbunden sein können, welches dadurch gekennzeichnet ist, dass die Umsetzung elektrochemisch durchgeführt wird.

Benachbarte Reste im Sinne der vorliegenden Erfindung sind solche Reste, die an direkt benachbarten C-Atomen gebunden sind. Im Falle der Verbindung (A) z. B. die Reste R¹ und R². Beispiele für solche Reste sind z.B. -O-(CH₂)_{z}-O- mit z = 1 bis 5, vorzugsweise 1 bis 3, bevorzugt 1

Die Verbindungen der Formel ABA stellen symmetrische Verbindungen aus der Gruppe der m-Terphenyle (nachfolgend gegebenenfalls auch m-Terphenylverbindungen oder symmetrische Pincerliganden, insbesondere NCN- oder OCO-Pincerliganden genannt) dar.

Als Terpheyle werden Diphenylbenzole bezeichnet. Als m-Terphenylen werden üblicherweise 1,3-Diphenylbenzole bezeichnet. Verbindungen aus der Gruppe der m-Terphenyle enthalten folglich eine 1,3-Diphenylbenzolleitstruktur. Im Rahmen der vorliegenden Erfindung versteht man unter symmetrischen Pincerliganden Verbindungen, die eine 1,3-Diphenylbenzoleinheit aufweisen, wobei die beiden äußeren Ringe keine Arene, sondern Phenole oder Aniline bzw. Phenol- oder Anilinderivate sind. Die OH- bzw. NR₂-Gruppen der beiden Phenylbausteine sind jeweils ortho zu der Arenkomponente angeordnet.

Unter symmetrischen Pincerliganden versteht man im Rahmen der vorliegenden Erfindung Verbindungen, bei denen die beiden Phenole bzw. Aniline identisch sind. Im Rahmen der Anmeldung muss die Arenkomponenten, die zwischen den beiden verschiedenen Phenylkomponenten angeordnet ist, nicht zwingend symmetrisch sein, d.h. es ist nicht notwendig, dass man, wenn man eine Spiegelebene in das Arenmolekül legt, die eine Seite auf die andere Seite abbildbar ist. Auch wenn die Arenkomponente an sich unsymmetrisch ist, ist das Produkt, also der resultierende Pincerligand, im Rahmen der Anmeldung als symmetrisch anzusehen.

Weiterhin wird im Rahmen der vorliegenden Erfindung folgende Nummerierung der Stellungen in der Arenkomponente (B) verwendet: 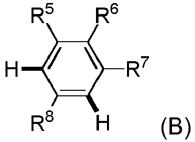
R⁵ ist definiert als Substituent am C-Atom 1 des Benzolringes, R⁶ als Substituent am C-Atom 2, R⁷ als Substituent am C-Atom 3, R⁸ als Substituent am C-Atom 5.

Die klassische Synthese von OCO-Pincerliganden ist bisher auf symmetrische Derivate beschränkt. Die bekannten Verfahren ermöglichen die C_{Ar}-C_{Ar}-Bindungsknüpfung beispielsweise durch Ullmann-Kupplung (B. G. Pring, Acta Chem. Scand. 1973, 27, 3873-3880), Grignard-Wurtz-Kupplung (R. S. Grewal, H. Hart, T. K. Vinod, J. Org. Chem. 1992, 57, 2721-2726.), oder Palladium-katalysierten Kupplungsreaktionen (S. Sarkar, A. R. Carlson, M. K. Veige, J. M. Falkowski, K. A. Abboud, A. S. Veige, J. Am. Chem. Soc. 2008, 130, 1116-1117). Vor den eigentlichen Kupplungsreaktionen ist der Schutz der Hydroxyfunktion notwendig, welche in den meisten Fällen mittels Methylierung erreicht wird. In allen bekannten Fällen ist die Durchführung der Reaktion unter Feuchtigkeitsausschluss und anaeroben Bedingungen notwendig.

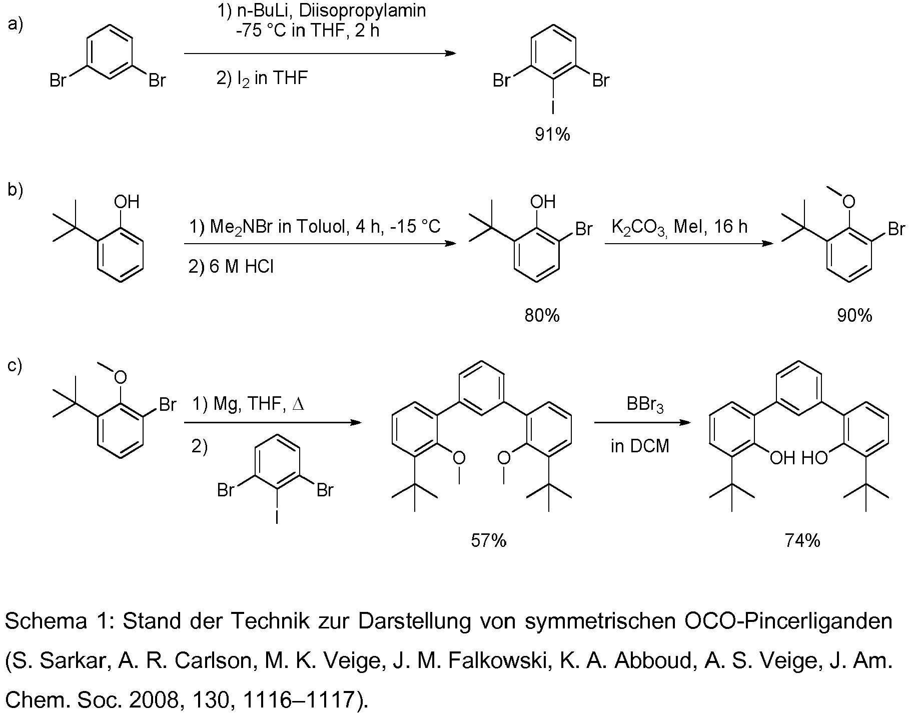

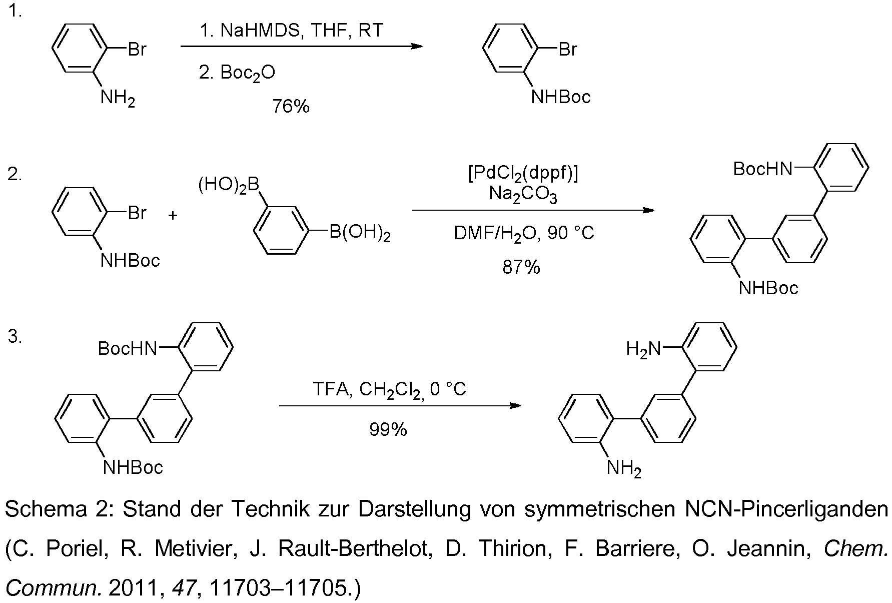

Ein großer Nachteil der bekannten Methoden zur Kreuzkupplung von Anilin- oder Phenolderivaten mit Arenen ist die Notwendigkeit trockener Lösungsmittel und eines Luftausschlusses während der Kupplungsreaktion. Bei klassischen Kreuzkupplungsmethoden ist der Schutz der Amin- bzw. Hydroxyfunktionalität in den meisten Fällen notwendig. Weiterhin werden für eine regioselektive Kreuzkupplung Abgangsfunktionalitäten in den eingesetzten Substraten eingeführt, die nach der eigentlichen Kupplungsreaktion als umweltproblematische Reaktionsabfälle anfallen (siehe Schema 1). Die Toleranz funktioneller Gruppen wird durch die eingesetzten Reagenzien oft eingeschränkt, so dass vor allem wenig substituierte Derivate eingesetzt werden können. Während der Reaktion treten oft toxische Nebenprodukte auf (wie beispielsweise Bromverbindungen), die vom gewünschten Produkt aufwendig abgetrennt und teuer entsorgt werden müssen. Ferner muss das Produkt über sehr viele Stufen hergestellt werden, was ebenfalls sehr unvorteilhaft ist (siehe Schema 1 und 2). Knapper werdende Rohstoffe machen die Darstellung von Ligandensystemen durch Palladium katalysierte Kreuzkupplungen oder chemische Oxidationsmittel sehr kostspielig.

Die Darstellung symmetrischer Pincerliganden ist klassisch sehr aufwändig. Man benötigt viele verschiedenen Reagenzien und Lösungsmittel und produziert darüber hinaus sehr viele Abfälle, die aus ökologischer und ökonomischer Sicht vermieden werden sollten. Aufgabe der vorliegenden Erfindung war deshalb die Bereitstellung eines einfachen Verfahrens zur Herstellung von symmetrischen Pincerliganden aus der Gruppe der m-Terphenylverbindungen bereitzustellen, welches einen oder mehrere Nachteile des Standes der Technik vermeidet.

Überraschenderweise wurde gefunden, dass diese Aufgabe dadurch gelöst werden kann, dass die Kupplungsreaktion elektrochemisch durchgeführt wird.

Gegenstand der vorliegenden Erfindung ist deshalb ein Verfahren wie in den Ansprüchen beansprucht.

Ebenfalls Gegenstand der vorliegenden Erfindung sind Verbindungen der Formel CDC wie nachfolgend definiert.

Außerdem ist Gegenstand der vorliegenden Erfindung die Verwendung der erfindungsgemäßen Verbindungen als Ligand zur Herstellung von Metall-Ligand-Katalysatorsystemen.

Das erfindungsgemäße Verfahren hat den Vorteil, dass durch die elektrochemische Umsetzung die Herstellung von symmetrischen m-Terphenylverbindungen wesentlich vereinfacht wird, da ein Feuchtigkeitsausschluss oder die Einhaltung von anaeroben Reaktionsbedingungen nicht gewährleistet werden muss. Weiterhin verläuft die Synthese direkt über eine Stufe und es können eine Vielzahl von Syntheseschritten eingespart werden. Dies führt unter anderem zu einem geringeren Aufwand bei der Durchführung des Verfahrens und zu einem Gesamtverfahren, das deutlich einfacher und damit wirtschaftlicher ist.

Die elektrochemische Umsetzung hat außerdem den Vorteil, dass die Bildung von unerwünschten Nebenprodukten deutlich reduziert werden kann.

Durch die elektrochemische Kreuzkupplung entfällt der Einsatz teurer und giftiger Katalysatoren und die Einführung aktivierender Abgangsfunktionalitäten, wie z. B. Bor oder Brom-Verbindungen, vor der Kupplungsreaktion kann entfallen.

Je nach Ausführungsart des erfindungsgemäßen Verfahrens erlaubt das Verfahren erstmals die direkte Synthese symmetrischer Verbindungen bei geringem synthetischem Aufwand und gleichzeitig hoher Toleranz funktioneller Gruppen in den eingesetzten Substraten.

Des Weiteren kann bei dem erfindungsgemäßen Verfahren teilweise oder vorzugsweise vollständig auf den Einsatz von chemischen Oxidationsmitteln verzichtet werden. Auf diese Weise wird die Gefahr der Bildung von Nebenprodukten bzw. der Verunreinigung der Verbindungen durch Rückstände der Oxidationsmittel vermieden.

Das erfindungsgemäße Verfahren hat außerdem den Vorteil, dass eine große Vielfalt unterschiedlicher m-Terphenylverbindungen dargestellt werden können, die nach den aus dem Stand der Technik bekannten Verfahren nicht oder nur unter großem Aufwand zugänglich waren.

Insbesondere hat das erfindungsgemäße Verfahren den Vorteil, dass mit ihm die erfindungsgemäßen m-Terphenylverbindungen (m-Terphenyle) hergestellt werden können.

Das erfindungsgemäße Verfahren, die erfindungsgemäßen Verbindungen sowie deren Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Werden nachfolgend Angaben in Prozent gemacht, so handelt es sich, wenn nicht anders angegeben um Angaben in Vol-%. Werden nachfolgend Mittelwerte angegeben, so handelt es sich, wenn nicht anders angegeben, um das Zahlenmittel. Werden nachfolgend Stoffeigenschaften, wie z. B. Viskositäten oder ähnliches angegeben, so handelt es sich, wenn nicht anders angegeben, um die Stoffeigenschaften bei 25 °C.

Das erfindungsgemäße Verfahren zur Herstellung von Verbindungen der Formel ABA 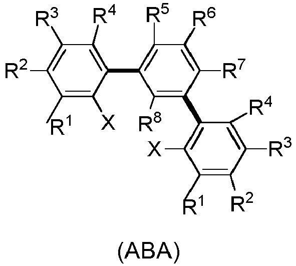 durch Umsetzen einer Verbindung der Formel (A) 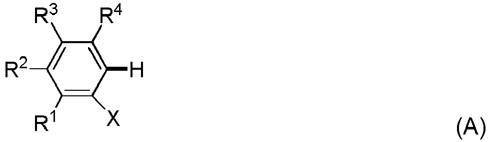 mit einer Verbindung der Formel (B) 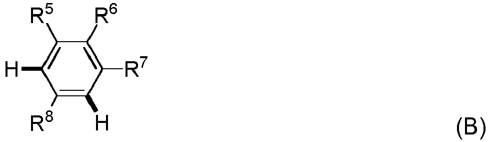
mit X = -OH oder -NR'R";
und R' = H oder eine Schutzgruppenfunktion, vorzugsweise ausgewählt aus der Gruppe umfassend *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl;
und R" = H oder eine Schutzgruppenfunktion, vorzugsweise ausgewählt aus der Gruppe umfassend *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl;
oder R' und R" miteinander kovalent verknüpfte Schutzgruppenreste, vorzugsweise Reste der Formeln PGa oder PGb 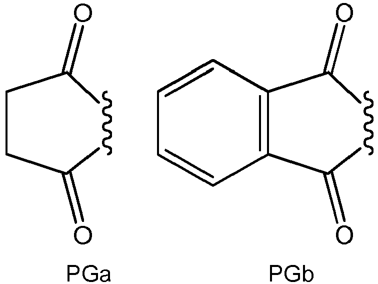
und R¹, R², R³, R⁴, R⁶ und R⁸ unabhängig voneinander ausgewählt sind aus der Gruppe enthaltend Wasserstoff, (C₁-C₁₂)-Alkyl, -O-(C₁-C₁₂)-Alkyl, -N[(C₁-C₁₂)-Alkyl]₂, -NH(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, -O-(C₁-C₁₂)-Alkyl, -N[(C₁-C₁₂)-Alkyl]₂ und Halogen, bevorzugt bestehend aus Wasserstoff, (C₁-C₁₂)-Alkyl und -O-(C₁-C₁₂)-Alkyl;
und R⁵ und R⁷ unabhängig voneinander ausgewählt sind aus der Gruppe enthaltend (C₁-C₁₂)-Alkyl, -O-(C₁-C₁₂)-Alkyl, -N[(C₁-C₁₂)-Alkyl]₂, -NH(C₁-C₁₂)-Alkyl, (C₆-C₂₀)-Aryl, -O-(C₆-C₂₀)-Aryl, Halogen, vorzugsweise bestehend aus (C₁-C₁₂)-Alkyl, -O-(C₁-C₁₂)-Alkyl, -N[(C₁-C₁₂)-Alkyl]₂ und Halogen, und bevorzugt bestehend aus (C₁-C₁₂)-Alkyl, -O-(C₁-C₁₂)-Alkyl und -N[(C₁-C₁₂)-Alkyl]₂;
wobei benachbarte Reste aus der Gruppe der Reste R¹ bis R⁸ über eine kovalente Bindung verbunden sein können,
dadurch gekennzeichnet, dass die Umsetzung elektrochemisch durchgeführt wird.

Im Rahmen der vorliegenden Erfindung wird unter Halogen Fluor, Chlor, Brom oder Iod verstanden, insbesondere Fluor, Chlor und Brom. Unter dem Begriff Alkyl wird im Rahmen der vorliegenden Erfindung ein linearer oder verzweigter Kohlenwasserstoffrest verstanden, bei dem gegebenenfalls ein oder mehrere Wasserstoffatome, vorzugsweise durch Halogenatome oder Hydroxyl- oder Alk(yl)oxy-Gruppen, substituiert sein können. Aryl bedeutet im Rahmen der vorliegenden Erfindung aromatische Kohlenwasserstoffreste, wie z. B. Phenyl- (C₆H₅-), Naphthyl- (C₁₀H₇-), Anthryl- (C₁₄H₉-), vorzugsweise Phenyl, bei denen gegebenenfalls ein oder mehrere Wasserstoffatome, vorzugsweise durch Alkyl- oder Alk(yl)oxy-Gruppen, substituiert sein können.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl-,oder Dodecyl-.

Die Reste "(C₁-C₁₂)-Alkyl und O-(C₁-C₁₂)-Alkyl" können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen

Der Ausdruck -(C₆-C₂₀)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, - CO-(C₆-C₂₀)-Aryl, -COOH, -OH, -SO₃H; -SO₃Na, -NO₂, -CN, -NH₂, -N[(C₁-C₁₂)-Alkyl]

Die elektrochemische Umsetzung erfolgt vorzugsweise so, dass
aa) eine Mischung mindestens eines Lösemittels und mindestens eines Leitsalzes erzeugt wird,
bb) zu dieser Mischung die umzusetzenden Verbindungen gegeben werden, wobei die Verbindung der Formel (A) vorzugsweise im molaren Überschuss bezogen auf die Verbindung (B) zugegeben wird,
cc) Einbringen von mindestens zwei Elektroden in die unter bb) erhaltene Reaktionslösung und Anlegen einer Spannung an die Elektroden,
dd) Umsetzen der Verbindungen (A) und (B) zu der Verbindung (ABA),
ee) Abschalten der Spannung, und ggf.
ff) Isolierung und/oder Aufreinigung der Verbindung (ABA).

In dem erfindungsgemäßen Verfahren werden die Verbindungen (A) und (B) vorzugsweise in solchen Mengen eingesetzt, dass das molare Verhältnis der Verbindung (A) zur Verbindung (B) im Bereich von 1,5 : 1 bis 4 : 1, bevorzugt von 1,8 : 1 bis 2,5 : 1 und besonders bevorzugt bei 2 : 1 beträgt.

In einer bevorzugten Ausführungsform des erfindungsgemäßen Verfahrens werden als Verbindungen der Formel (A) solche Verbindungen eingesetzt, bei denen X = -NR'R" mit R' und R" unabhängig voneinander ausgewählt sind aus der Gruppe umfassend Wasserstoff, *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl, bevorzugt bestehend aus Wasserstoff, *tert*-Butyloxycarbonyl, Methyloxycarbonyl, Benzyloxycarbonyl, Phenyloxycarbonyl, Acetyl, Trifluoracetyl und Benzoyl, und besonders bevorzugt bestehend aus Wasserstoff, Acetyl und Trifluoracetyl.
oder R' und R" miteinander kovalent verknüpft, vorzugsweise über eine der nachfolgenden Gruppen PGa oder PGb 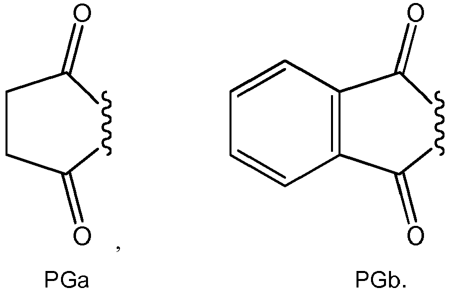 Bevorzugt ist einer der Reste R' oder R" = H. Besonders bevorzugt X = -NH₂.

Werden in dem erfindungsgemäßen Verfahren solche Verbindungen der Formel (A) eingesetzt, bei denen R' oder R" ungleich H ist, so können solche Verbindungen auf bekannte Weise aus den Verbindungen der Formel (A) mit R' und/oder R" = H durch Einführen einer Schutzgruppe erhalten werden. Schutzgruppen und deren Einführung sind dem Fachmann bekannt und kann der gängigen Fachliteratur entnommen werden (siehe hierzu auch: P. G. M. Wuts, T. W. Greene "Greenes's Protective Groups in Organic Synthesis", fourth edition, 2007, John Wiley and Sons; Hoboken, New Jersey)

In einer weiteren bevorzugten Ausführungsart des erfindungsgemäßen Verfahrens wird als Verbindung der Formel (A) vorzugsweise eine Verbindung (A) eingesetzt, bei der X = -NH₂ ist.

In einer weiteren bevorzugten Ausführungsart des erfindungsgemäßen Verfahrens wird als Verbindung der Formel (A) vorzugsweise eine Verbindung (A) eingesetzt, bei der X = -OH ist.

In dem erfindungsgemäßen Verfahren werden vorzugsweise solche Verbindungen der Formel (B) eingesetzt, bei denen R⁶ und R⁸ identisch sind und bevorzugt Wasserstoff bedeuten.

In dem erfindungsgemäßen Verfahren werden vorzugsweise solche Verbindungen der Formel (B) eingesetzt, bei denen R⁵ und R⁷ verschieden sind und bevorzugt (C₁-C₁₂)-Alkyl, O-(C₁-C₁₂)-Alkyl oder -N[(C₁-C₁₂)-Alkyl]₂ bedeuten.

Bevorzugt werden in dem erfindungsgemäßen Verfahren solche Verbindungen der Formel (B) eingesetzt, bei denen R⁶ und R⁸ identisch sind und bevorzugt Wasserstoff bedeuten und bei denen R⁵ und R⁷ verschieden sind und bevorzugt (C₁-C₁₂)-Alkyl, O-(C₁-C₁₂)-Alkyl oder -N[(C₁-C₁₂)-Alkyl]₂ bedeuten.

Die erfindungsgemäß durchgeführte elektrochemische Umsetzung kann z. B. in Anlehnung an die von a.) A. Kirste, B. Elsler, G. Schnakenburg, S. R. Waldvogel, J. Am. Chem. Soc. 2012, 134, 3571-3576, b.) A. Kirste, S. Hayashi, G. Schnakenburg, I. M. Malkowsky, F. Stecker, A. Fischer, T. Fuchigami, S. R. Waldvogel, Chem. Eur. J. 2011, 17, 14164-14169, c.) B. Elsler, D. Schollmeyer, K. M. Dyballa, R. Franke, S. R. Waldvogel, Angew. Chem. Int. Ed. 2014, 53, 5210-5213, und d.) A. Kirste, M. Nieger, I. M. Malkowsky, F. Stecker, A. Fischer, S. R. Waldvogel, Chem. Eur. J. 2009, 15, 2273-2277 beschriebenen Verfahren zur elektrochemischen Kupplung von Kohlenstoff-Kohlenstoff-Bindungen, durchgeführt werden.

Die elektochemische Umsetzung kann in allen geeigneten, aus dem Stand der Technik bekannten Elektrolysezellen durchgeführt werden. Bevorzugt wird in dem erfindungsgemäßen Verfahren eine Flanschzelle, eine Becherglaszelle oder eine Screeningzelle eingesetzt. Solche Zellen sind literaturbeschrieben und die ersten beiden Glaszellen können, z.B. unter dieser Bezeichnung bei HWS Labortechnik Mainz bezogen werden.

Die Zelle weist mindestens zwei Elektroden auf. Als Elektroden können handelsübliche Elektroden eingesetzt werden. Als Anoden können vorzugsweise z. B. BDD (0,015 mm bor-dotierter Diamant auf Silizium oder 0,05 mm bor-dotiertem Diamant auf Niob), Platin-, isostatische Graphit- oder Glaskohlenstoffelektroden eingesetzt werden. Als Kathoden werden vorzugsweise BDD-, Nickelnetz- oder Glaskohlenstoffelektroden eingesetzt. Solche BDD-Elektroden (bor-dotierter Diamant auf einem Träger, wie z.B. Niob oder Silizium) sind z. B. unter der Bezeichnung DIACHEM bei der Firma Condias GmbH Itzehoe erhältlich. Die restlichen Elektroden sind von gängigen Chemikalien- und Materiallieferanten, wie z.B. Goodfellow oder Aldrich erhältlich.

Die elektrochemische Umsetzung wird vorzugsweise in Gegenwart eines Lösemittels durchgeführt. Als Lösemittel wird vorzugsweise ein Lösemittel aus der Gruppe von Acetonitril, Propylencarbonat, Methylcarbonat, Nitromethan, Ethylenglycoldimethylether, Methansulfonsäure, Benzol, Toluol, Wasser, Methanol, Ethanol, Propanol, Isopropanol, halogenierte Lösemittel oder halogenierte oder nicht halogenierte Säuren oder Mischungen dieser eingesetzt.

Bevorzugt werden als Lösemittel eine Carbonsäure, vorzugsweise Ameisensäure, zugegeben, eine fluorierte Carbonsäure oder ein fluorierter Alkohol, vorzugsweise Trifluoressigsäure oder 1,1,1,3,3,3-Hexafluor-2-propanol (HFIP), bevorzugt 1,1,1,3,3,3-Hexafluor-2-propanol eingesetzt.

Besonders bevorzugt wird als Lösemittel Methanol, Ameisensäure Trifluoressigsäure, Hexafluorisopropanol oder Mischungen dieser, vorzugsweise Methanol, Hexafluorisopropanol oder Mischungen dieser, und besonders bevorzugt Hexafluorisopropanol (1,1,1,3,3,3-Hexafluor-2-propanol) verwendet.

Der elektrochemische Umsetzung wird vorzugsweise in Gegenwart mindestens eines Leitsalzes durchgeführt wird, wobei als Leitsalze bevorzugt solche eingesetzte werden, die ausgewählt sind aus der Gruppe von Tetra(C₁-C₆-alkyl)-ammonium- oder 1,3-Di(C₁-C₆-alkyl)imidazoliumsalzen, mit der Maßgabe, dass die Alkylgruppen Halogen-substituiert, insbesondere mit Fluor-substituiert sein können. Vorzugsweise werden solche Leitsalze eingesetzt, die Gegenionen aufweisen, die ausgewählt sind aus der Gruppe umfassend Arsenat, Sulfat, Hydrogensulfat, Alkylsulfat, Alkylphosphat, Perchlorat, Fluorid, Arylsulfat, Hexafluorphosphat und Tetrafluorborat. Bevorzugte Leitsalze sind solche aus der Gruppe von quaternären Ammoniumborate, Ammoniumfluoralkylphosphate, Ammoniumfluoralkylarsenate, Ammoniumtrifluor-methylsulfonate, Ammoniumbis(fluoromethansulfon)imide, Ammoniumtris- (fluoromethansulfonyl)methide, Methyltributylammoniummethylsulfat, Methyltriethylammoniummethylsulfat, Tetrabutyl-ammoniumhexafluorophosphat, Tetraethylammoniumtetrafluoroborat, Lithiumhexafluorophosphat oder Tetraethylammoniumtetrafluoroborat. Besonders bevorzugt wird Methyltriethylammoniummethylsulfat oder Bu₃NMe⁺MeOSO₃⁻ (Methyltributylammoniummethylsulfat), ganz besonders bevorzugt Methyltributylammoniummethylsulfat als Leitsalz eingesetzt.

Das Leitsalz wird vorzugsweise in einer Konzentration von 0,001 bis 10 mol/L, bevorzugt 0,01 bis 1 mol/L, besonders bevorzugt von 0,075 bis 0,125 mol/L und ganz besonders bevorzugt von 0,09 mol/L bezogen auf die Reaktionsmischung eingesetzt.

Vorzugsweise wird in dem erfindungsgemäßen Verfahren jeweils so viel an Verbindung A eingesetzt, dass die Konzentration von 0,001 bis 5 mol/L, vorzugsweise von 0,05 bis 0,5 mol/L, bevorzugt von 0,1 bis 0,3 mol/L und besonders bevorzugt 0,15 mol/L Reaktionsmischung beträgt.

Die elektrochemische Umsetzung wird vorzugsweise bei Raumtemperatur oder erhöhter Temperatur durchgeführt. Bevorzugt wird die elektrochemische Umsetzung bei einer Temperatur in der Elektrolysezelle im Bereich von 25 bis 80 °C, bevorzugt von 35 bis 70 °C und bevorzugt von 45 bis 55 °C durchgeführt.

Die elektrochemische Umsetzung wird vorzugsweise galvanostatisch durchgeführt.

Bei der Durchführung der elektrochemischen Umsetzung wird die Stromstärke vorzugsweise so gewählt, dass die Stromdichte von 1 bis 10 mA/cm², vorzugsweise von 2 bis 5,5 mA/cm², vorzugsweise von 2,5 bis 3 mA/cm² und bevorzugt 2,8 mA/cm² beträgt. Zur Durchführung der elektrochemischen Umsetzung wird vorzugsweise eine (Klemm-)Spannung zwischen den Elektroden im Bereich von 2 bis 10 V, bevorzugt 2,5 bis 7,5 V und bevorzugt von 3 bis 6 V angelegt.

Nachfolgend wird durch zwei Reaktionsschemata beispielhaft die Durchführung des erfindungsgemäßen Verfahrens aufgezeigt. Die in den Schemen verwendeten Reste R¹ bis R⁸ sind wie oben definiert. Die Kupplung eines substituierten Phenols an ein 1,3-disubstituiertes Aren, welches in 2 und 5 Stellung ebenfalls substituiert sein kann, führt zu einer Verbindung, die der Formel ABA.

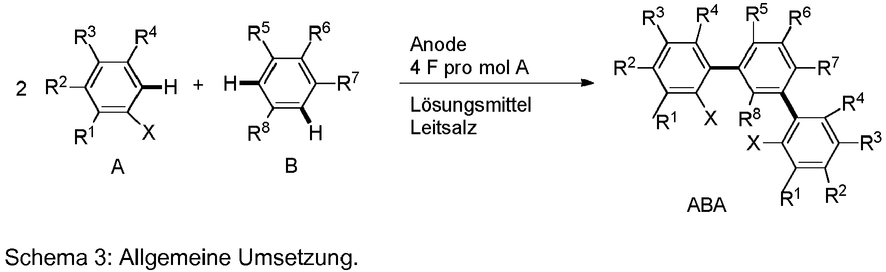

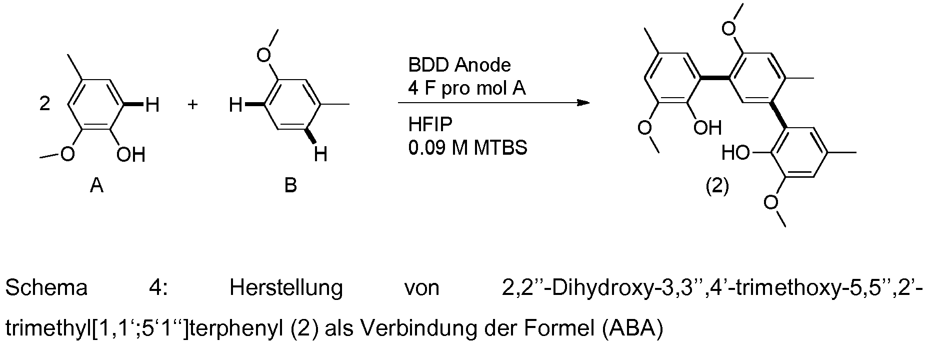

Solche Verbindungen sind auf klassischem Wege bisher nicht beschrieben bzw. nur mit hohem synthetischen Aufwand zugänglich.

Das erfindungsgemäße Verfahren wird vorzugsweise so durchgeführt, dass die Umsetzung in den elektrochemischen Verfahrensschritten, bevorzugt die gesamte Umsetzung ohne die Verwendung von organischen Oxidationsmitteln durchgeführt wird.

Mit dem erfindungsgemäßen Verfahren können insbesondere die erfindungsgemäßen Verbindungen der Formel (CDC) hergestellt werden.

Die erfindungsgemäßen Verbindungen der Formel (CDC) 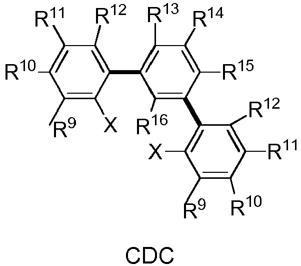
X = -OH oder -NR'R"
mit R' oder R" unabhängig voneinander ausgewählt aus der Gruppe umfassend Wasserstoff, *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl, vorzugsweise bestehend aus Wasserstoff, *tert*-Butyloxycarbonyl, Methyloxycarbonyl, Benzyloxycarbonyl, Phenyloxycarbonyl, Acetyl, Trifluoracetyl und Benzoyl, bevorzugt bestehend aus Wasserstoff, Acetyl und Trifluoracetyl;
und besonders bevorzugt sind R' und R" Wasserstoff, oder R' und R" sind miteinander kovalent verknüpft, vorzugsweise über eine der nachfolgenden Gruppen PGa oder PGb 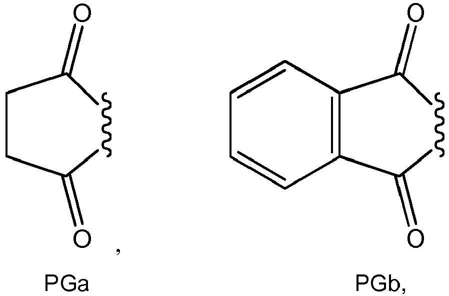
und R⁹, R¹⁰, R¹¹ und R¹² unabhängig voneinander ausgewählt sind aus der Gruppe enthaltend Wasserstoff, (C₁-C₁₂)-Alkyl, O-(C₁-C₁₂)-Alkyl, -N[(C₁-C₁₂)-Alkyl]₂, -NH(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, O-(C₁-C₁₂)-Alkyl, -N[(C₁-C₁₂)-Alkyl]₂ und Halogen, bevorzugt bestehend aus Wasserstoff, (C₁-C₁₂)-Alkyl, O-(C₁-C₁₂)-Alkyl und -N[(C₁-C₁₂)-Alkyl]₂;
und R¹³ und R¹⁵ unabhängig voneinander ausgewählt sind aus der Gruppe enthaltend (C₁-C₁₂)-Alkyl, O-(C₁-C₁₂)-Alkyl, -N[(C₁-C₁₂)-Alkyl]₂;
und R¹⁴ und R¹⁶ Wasserstoff sind,
wobei benachbarte Reste aus der Gruppe der Reste R⁹ bis R¹² über eine kovalente Bindung verbunden sein können.

Vorzugsweise ist in den erfindungsgemäßen Verbindungen der Formel (CDC) X = -OH oder -NH₂, bevorzugt -OH.

Bevorzugte erfindungsgemäße Verbindungen der Formel (CDC) sind solche, die den Formeln (1) und (2) 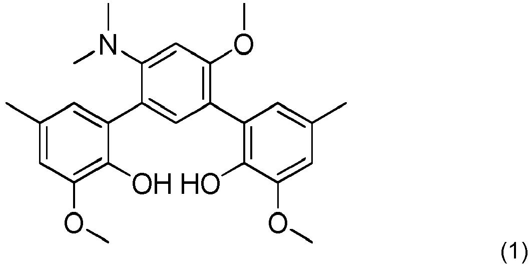 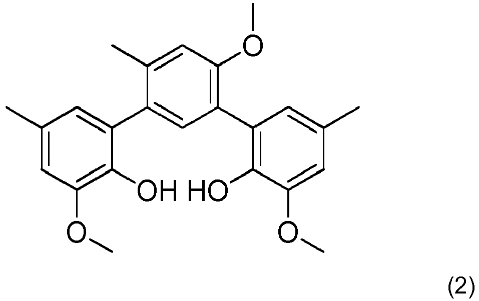 genügen.

Die erfindungsgemäßen Verbindungen können als Ligand zur Herstellung von Metall-Ligand-Katalysatorsystemen verwendet werden.

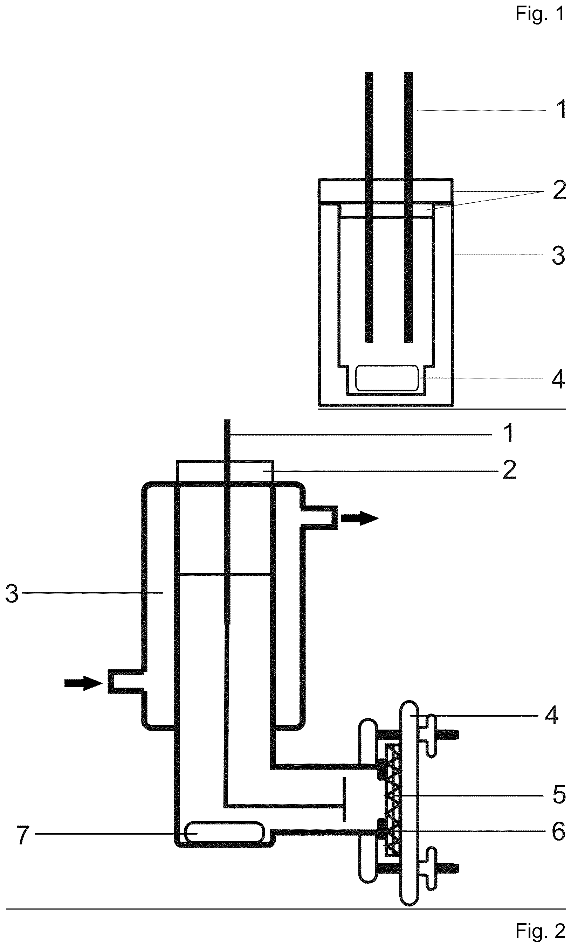

Die vorliegende Erfindung wird an Hand der Figuren 1 bis 4 beispielhaft beschrieben, ohne dass die Erfindung, deren Anwendungsbreite sich aus der gesamten Beschreibung und den Ansprüchen ergibt, auf die in den Figuren abgebildeten Ausführungsformen beschränkt sein soll. In den Figuren 1 bis 4 sind schematische Zeichnungen von Zellen, wie sie im erfindungsgemäßen Verfahren eingesetzt werden können und wie sie insbesondere in den nachfolgenden Beispielen verwendet wurden, abgebildet.
Fig. 1 zeigt schematisch eine Screening-Zelle. In Fig. 2 sind mit 1 die BDD-Elektroden, mit 2 ein Teflon-Deckel, mit 3 ein Teflon-Becher und mit 4 ein Rührmagnet gekennzeichnet.
Fig. 2 zeigt schematisch eine Flanschzelle (Glas). In Fig. 2 sind mit 1 ein Edelstahl-Stab mit befestigter Nickel-Netz-Kathode, mit 2 ein Teflon-Stopfen, mit 3 ein Kühlmantel (Glas), mit 4 eine Schraubzwinge, mit 5 eine BDD-Elektrode, mit 6 eine EPDM Dichtung und mit 7 ein Rührmagnet gekennzeichnet.

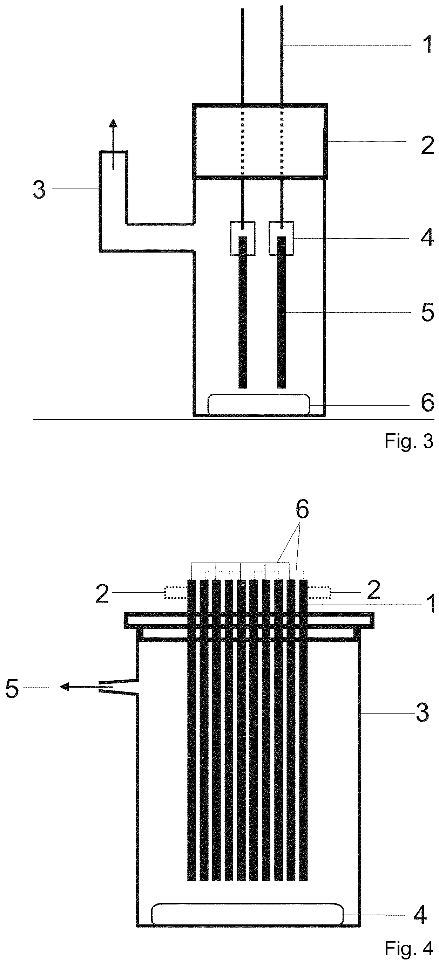

Fig. 3 zeigt schematisch eine Becherglas-Zelle klein (Glas). In Fig. 3 sind mit 1 ein Edelstahl-Stab zur Kontaktierung, mit 2 ein Teflon-Stopfen, mit 3 ein Zellen-Auslass/Anschluss, z. B. für einen Dimroth-Kühler, mit 4 Elektrodenhalterungen (Edelstahl), mit 5 Glaskohlenstoff-Elektroden und mit 6 ein Rührmagnet gekennzeichnet.
Fig. 4 zeigt schematisch eine Becherglaszelle groß (Glas). In Fig. 4 sind mit 1 die BDD-Elektroden, mit 2 die Anschlüsse zur Kontaktierung, mit 3 der Glasmantel, mit 4 ein Rührmagnet, mit 5 ein Zellen-Auslass/Anschluss, z.B. für einen Dimroth-Kühler und mit 6 ein elektrisch leitender Kontakt (Edelstahlfolie) bezeichnet.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Experimenteller Teil

### Allgemeine Methoden

### Chromatographie (GC/GCMS)

Präparative Flüssigchromatographie zur Auftrennung von Stoffgemischen wurde unter Verwendung von Kieselgel 60 M (0,040-0,063 mm) der Firma MACHERY-NAGEL GMBH & CO. KG, Düren bei einem Maximaldruck von 2 bar durchgeführt. Alle verwendeten Eluenten (Essigsäureethylester, technische Qualität; Cyclohexan, technische Qualität) wurden vorab destillativ am Rotationsverdampfer gereinigt.

Dünnschichtchromatographie (DC) wurde an PSC-Fertigplatten Kieselgel 60 F₂₅₄ der Firma MERCK KGaA, Darmstadt durchgeführt. Detektion der verschiedenen Substanzen erfolgte zunächst unter UV-Licht und anschließend durch Anfärben mittels Cer-Molybdatophosphorsäure-Reagenz (5,6 g Molybdatophosphorsäure, 2,2 g Cer(IV)-sulfat-Tetrahydrat und 13,3 g konz. Schwefelsäure auf 200 mL Wasser) und anschließendem Erhitzen durch einen Heißluftfön.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0,25 mm; Filmdicke der kovalent gebundenen stationären Phase: 0,25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0,25 mm; Filmdicke der kovalent gebundenen stationären Phase: 0,25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma Thermo Finnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCI3 verwendet. Die ¹H- und ¹³C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht zwingend mit der IUPAC-Nomenklatur übereinstimmen muss.

### Einkristallstrukturanalysen

Die Einkristallstrukturanalysen wurden im Institut für Organische Chemie der Johannes Gutenberg-Universität Mainz an einem Gerät des Typs IPDS 2T der Firma STOE & Cie GmbH, Darmstadt durchgeführt.

### Schmelzpunkte

Die relevanten Schmelzpunkte wurden mit Hilfe des Schmelzpunktbestimmungsgerätes SG 2000 der Firma HW5, Mainz gemessen und wurden unkorrigiert übernommen.

### Allgemeine Arbeitsvorschriften

### AAV1: Elektrochemische Kreuzkupplung in L-Zellen

5 mmol der zu oxidierenden Spezies A (Unterschusskomponente) wurden mit einem 2-3 fachen Überschuss (10-15 mmol) des Kupplungspartners B in 33 mL 1,1,1,3,3,3-Hexafluorisopropanol (HFIP) bzw. 33 mL (HFIP mit 18 Vol% Methanol (MeOH) bezogen auf die Summe aus HFIP und MeOH) in einer ungeteilten Flanschzelle mit BDD-Anode und Nickelnetzkathode umgesetzt. Als Leitsalz verwendete man Bu₃NMe⁺MeOSO₃⁻ (MTBS) mit einer Konzentration von 0.09 M. Die Elektrolyse erfolgte galvanostatisch. Der Außenmantel der Elektrolysezelle temperierte man über einen Thermostaten auf etwa 10 °C, während die Reaktionsmischung gerührt und mit Hilfe eines Ölbades auf 50 °C erhitzt wurde. Nach Ende der Elektrolyse wurde der Zellinhalt in einen 50 mL Rundhalskolben überführt und das Lösungsmittel unter vermindertem Druck am Rotationsverdampfer bei 50 °C, 200 - 70 mbar entfernt. Mineralisationsprodukte sowie das enthaltene Leitsalz trennte man durch Elution mittels Essigsäureethylester (300 mL) über 50 g Kieselgel 60 ab. Nicht umgesetztes Edukt wurde mittels Kurzwegdestillation an der Kugelrohrdestille zurückerhalten (100 °C, 10⁻³ mbar). Die entstandenen Reaktionsprodukte wurden wie jeweils angegeben säulenchromatographisch getrennt.

### Elektrodenmaterial:

| | |
|---|---|
| Anode: | BDD (15 µm Diamantschicht) auf Siliciumträger |
| Kathode: | Nickelnetz |

### Elektrolysebedingungen:

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3 - 6 V |

### Einstufige Pincerligandensynthese

### Beispiel 1:

### 2,2"-Dihydroxy-5,5"-dimethyl-2'-N,N-dimethylamin-3,3",4'-trimethoxy[1,1';5'1"]terphenyl (1)

Die Durchführung der Elektrolyse erfolgte gemäß der AAV1 mit 1,38 g (10 mmol, 2,0 Äquiv.) 4-Methylguajacol und 0,76 g (5 mmol, 1,0 Äquiv.) 3-(*N*,*N*-Dimethylamino)anisol. Die Stromstärke betrug 15 mA, die Ladungsmenge betrug mit 4 F pro 3-(*N*,*N-*Dimethylamino)anisol 1929,7 C. Das Rohprodukt wurde säulenchromatographisch an Kieselgel 60 mit einem Laufmittelgemisch-Gradienten von 95:5, dann 9:1 und schließlich 4:1 (Cyclohexan (CH): Ethylacetat (EE)) vorgereinigt. Die erhaltenen Fraktionen wurden auf Vorhandensein des Produktes geprüft und erneut an Kieselgel 60 mit einem Eluenten 1:1:0,01, dann 1:1:0,02 (Dichlormethan (DCM): Cyclohexan (CH): Methanol (MeOH)) gereinigt. Eine weitere Aufreinigung von Mischfraktionen wurde durch Kugelrohrdestillation (150 °C, 180 °C, 190 °C und 205 °C, 10⁻³ mbar) durchgeführt. Das Produkt wurde als gelbes Öl erhalten. 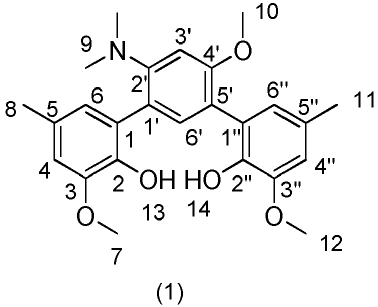
Ausbeute: 54 mg (0,13 mmol, 3%)
GC (Methode *hart,* HP-5): t_{R} = 18,40 min
R_{f} (DCM:MeOH = 99:1) = 0,36
¹H-NMR (300 MHz, CDCl₃) δ [ppm] = 2.28 (s, 3H, H-8 o. H-11), 2.29 (s, 3H, H-8 o. H-11), 2.85 (bs, 6H, H-9), 3.77 (s, 3H, H-10), 3.81 (s, 3H, H-7 o. H-12), 3.82 (s, 3H, H-7 o. H-12), 6.40 (bs, 1H, H-13 o. H-14), 6.57 (s, 2H, H-4, H-4"), 6.59-6.61 (m, 1H, H-6'), 6.67 (m, 1H, H-3'), 6.68-6.72 (m, 2H, H-6, H-6").

¹³C-NMR (101 MHz, CDCl₃) δ [ppm] = 21.10 (C-8 o. C-11), 21.22 (C-8 o. C-11), 43.44 (C-9), 55.71 (C-10), 55.81 (C-7 o. C-12), 56.91 (C-7 o. C-12), 104.68 (C-3'), 111.99, 112.07, 113.08, 113.26, 113.86, 116.59, 118.44, 120.83, 121.01, 121.50, 130.30, 132.23, 144.14, 146.66, 149.30 (C-3 o. C-3"), 150.03 (C-3 o. C-3"), 157.70 (C-4').

HRMS (ESI, pos. mode): *m*/*z* für C₂₅H₃₀NO₅ [M+H⁺]:
berechnet: 424,2124; gefunden: 424,2131

| | | | | |
|---|---|---|---|---|
| Elementaranalyse: | berechnet: | C: 70,90% | H: 6,90% | N: 3,31% |
| | gefunden: | C: 69,33% | H: 6,95% | N: 3,17% |

### Beispiel 2:

### 2,2"-Dihydroxy-3,3",4'-trimethoxy-5,5",2'-trimethy[1,1';5'1"]terphenyl (2)

Die Elektrolyse wurde gemäß AAV1 mit 1,45 g (10,5 mmol, 2,0 Äquiv.) 4-Methylguajakol und 639 mg (5,23 mmol, 1,0 Äquiv.) 3-Methylanisol durchgeführt. Die Stromdichte betrug 2,8 mA/cm², die Ladungsmenge 2 F pro 4-Methylguajakol (Q = 2018 C). Nach Abtrennung des Lösungsmittels wurde die Produktmischung säulenchromatographisch an Kieselgel 60 mit einem Eluenten von 9:1 (CH:EE) aufgereinigt. Das Produkt wurde als gelbliche, ölartige Substanz erhalten. 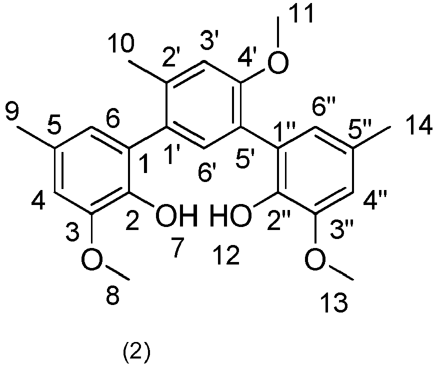
Ausbeute: 68 mg (0,17 mmol, 3%)
GC (Methode *hart,* HP-5): t_{R} = 21,35 min
R_{f} (Cy:EE = 4:1) = 0,39
¹H-NMR (400 MHz, CDCl₃) δ [ppm] = 2.28 (s, 3H, 14-H), 2.29 (s, 3H, 9-H), 2.30 (s, 3H, 10-H), 3.89 (s, 3H, 8-H), 3.90 (s, 3H, 13-H), 3.91 (s, 3H, 11-H), 6.00 (bs, 2H, 7-H, 12-H), 6.69-6.72 (m, 4H, 4-H, 4"-H, 6-H, 6"-H), 6.93 (s, 1H, 3'-H), 7.19 (s, 1H, 6'-H).

¹³C-NMR (101 MHz, CDCl₃) δ [ppm] = 20.33 (C-10), 21.23, 21.26 (C-9, C-14), 56.13, 56.14 (C-11, C-13), 56.17 (C-8), 110.72 (C-4), 111.56 (C-4"), 113.02 (C-3'), 123.69, 123.76 (C-6, C-6"), 124.44 (C-5'), 125.25,125.53 (C-1, C-1"), 128.88 (C-6'), 129.39 (C-1'), 130.62, 133.66 (C-5, C-5"), 138.05 (C-2'), 140.63, 141.07, 146.40 (C-3, C-3", C-4'), 147.66 (C-2), 155.37 (C-2").

HRMS (ESI, pos. mode): *m*/*z* für C₂₄H₂₆O₅ (M+Na⁺):
berechnet: 417,1678; gefunden: 417,1670
Schmelzpunkt: 67,7 °C (aus DCM kristallisiert)

## Patentansprüche

1. Verfahren zur Herstellung von Verbindungen der Formel (ABA) 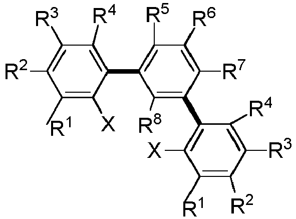 (ABA)
durch Umsetzen einer Verbindung der Formel (A) 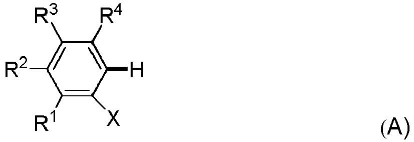 mit einer Verbindung der Formel (B) 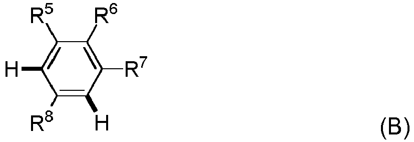
mit X = -OH oder -NR'R";
und R' = H oder eine Schutzgruppenfunktion, vorzugsweise ausgewählt aus der Gruppe umfassend *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl;
und R" = H oder eine Schutzgruppenfunktion, vorzugsweise ausgewählt aus der Gruppe umfassend *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl;
oder R' und R" miteinander kovalent verknüpfte Schutzgruppenreste, vorzugsweise Reste der Formeln PGa oder PGb 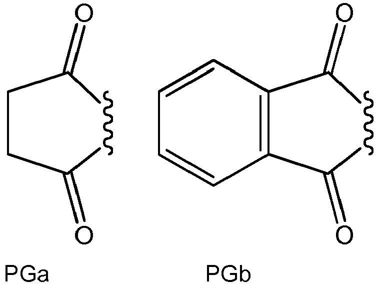
und R¹, R², R³, R⁴, R⁶ und R⁸ unabhängig voneinander ausgewählt sind aus der Gruppe enthaltend Wasserstoff, (C₁-C₁₂)-Alkyl, -O-(C₁-C₁₂)-Alkyl, -N[(C₁-C₁₂)-Alkyl]₂, -NH(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, -O-(C₁-C₁₂)-Alkyl, -N[(C₁-C₁₂)-Alkyl]₂ und Halogen, bevorzugt bestehend aus Wasserstoff, (C₁-C₁₂)-Alkyl und -O-(C₁-C₁₂)-Alkyl;
und R⁵ und R⁷ unabhängig voneinander ausgewählt sind aus der Gruppe enthaltend (C₁-C₁₂)-Alkyl, -O-(C₁-C₁₂)-Alkyl, -N[(C₁-C₁₂)-Alkyl]₂, -NH(C₁-C₁₂)-Alkyl, (C₆-C₂₀)-Aryl, -O-(C₆-C₂₀)-Aryl, Halogen, vorzugsweise bestehend aus (C₁-C₁₂)-Alkyl, -O-(C₁-C₁₂)-Alkyl, -N[(C₁-C₁₂)-Alkyl]₂ und Halogen, und bevorzugt bestehend aus (C₁-C₁₂)-Alkyl, -O-(C₁-C₁₂)-Alkyl und -N[(C₁-C₁₂)-Alkyl]₂;
wobei benachbarte Reste aus der Gruppe der Reste R¹ bis R⁸ über eine kovalente Bindung verbunden sein können,
**dadurch gekennzeichnet,**
**dass** die Umsetzung elektrochemisch durchgeführt wird.

2. Verfahren nach Anspruch 1,
**dadurch gekennzeichnet,**
**dass** der elektrochemische Verfahrensschritt so durchgeführt wird, dass
aa) eine Mischung mindestens eines Lösemittels und mindestens eines Leitsalzes erzeugt wird,
bb) zu dieser Mischung die umzusetzenden Verbindungen gegeben werden, wobei die Verbindung der Formel (A) im molaren Überschuss bezogen auf die Verbindung (B) zugegeben wird,
cc) Einbringen von mindestens zwei Elektroden in die unter bb) erhaltene Reaktionslösung und Anlegen einer Spannung an die Elektroden,
dd) Umsetzen der Verbindungen (A) und (B) zu der Verbindung (ABA),
ee) Abschalten der Spannung, und ggf.
ff) Isolierung und/oder Aufreinigung der Verbindung (ABA).

3. Verfahren nach einem der Ansprüche 1 oder 2,
**dadurch gekennzeichnet,**
**dass** das molare Verhältnis der Verbindung (A) zur Verbindung (B) im Bereich von 1,5 : 1 bis 4 : 1, bevorzugt von 1,8 : 1 bis 2,5 : 1 und besonders bevorzugt bei 2 : 1 liegt.

4. Verfahren nach einem der Ansprüche 1 bis 3,
**dadurch gekennzeichnet,**
**dass** als Verbindung der Formel (A) Verbindungen eingesetzt werden, bei denen X = -NHR' ist.

5. Verfahren nach einem der Ansprüche 1 bis 3,
**dadurch gekennzeichnet,**
**dass** als Verbindung der Formel (A) Verbindungen eingesetzt werden, bei denen X = -NH₂ ist.

6. Verfahren nach einem der Ansprüche 1 bis 5,
**dadurch gekennzeichnet,**
**dass** R⁶ und R⁸ identisch sind und vorzugsweise Wasserstoff bedeuten.

7. Verfahren nach einem Ansprüche 1 bis 6,
**dadurch gekennzeichnet,**
**dass** die Umsetzung in Gegenwart eines Lösemittels durchgeführt wird und als Lösemittel ein Lösemittel aus der Gruppe von Acetonitril, Propylencarbonat, Methylcarbonat, Nitromethan, Ethylenglycoldimethylether, Methansulfonsäure, Benzol, Toluol, Wasser, Methanol, Ethanol, Propanol, Isopropanol, halogenierte Lösemittel oder halogenierte oder nicht halogenierte Säuren oder Mischungen dieser eingesetzt wird.

8. Verfahren nach Anspruch 6,
**dadurch gekennzeichnet,**
**dass** als Lösemittel Methanol, Ameisensäure Trifluoressigsäure, Hexafluorisopropanol oder Mischungen dieser, vorzugsweise Methanol, Hexafluorisopropanol oder Mischungen dieser, und bevorzugt Hexafluorisopropanol verwendet wird.

9. Verfahren nach einem der Ansprüche 1 bis 8,
**dadurch gekennzeichnet,**
**dass** die Umsetzung ohne die Verwendung von organischen Oxidationsmitteln durchgeführt wird.

10. Verfahren nach einem der Ansprüche 1 bis 9,
**dadurch gekennzeichnet,**
**dass** der elektrochemische Verfahrensschritt in Gegenwart mindestens eines Leitsalzes durchgeführt wird, wobei als Leitsalze vorzugsweise solche eingesetzte werden, die ausgewählt sind aus der Gruppe von Tetra(C₁-C₆-alkyl)-ammonium- oder (1,3-Di(C₁-C₆-alkyl)imidazoliumsalzen, mit der Maßgabe, dass die alkylGruppen Halogen-substituiert sein können.

11. Verfahren nach Anspruch 9,
**dadurch gekennzeichnet,**
**dass** die Gegenionen der Leitsalze ausgewählt sind aus der Gruppe umfassend Arsenat, Sulfat, Hydrogensulfat, Alkylsulfat, Alkylphosphat, Perchlorat, Fluorid, Arylsulfat, Hexafluorphosphat und Tetrafluorborat.

12. Verfahren nach Anspruch 9 oder 10,
**dadurch gekennzeichnet,**
**dass** als Leitsalz ein Salz aus der Gruppe umfassend quaternäre Ammoniumborate, Ammoniumfluoralkylphosphate, Ammoniumfluoralkylarsenate, Ammoniumtrifluor-methylsulfonate, Ammoniumbis(fluoromethansulfon)imide, Ammoniumtris- (fluoromethansulfonyl)methide, Methyltributylammoniummethylsulfat, Methyltriethylammoniummethylsulfat, Tetrabutyl-ammoniumhexafluorophosphat, Tetraethylammoniumtetrafluoroborat, Lithiumhexafluorophosphat oder Tetraethylammoniumtetrafluoroborat, bevorzugt Methyltriethylammoniummethylsulfat oder Bu3NMe+MeOSO3-(Methyltributylammoniummethylsulfat) und besonders bevorzugt Methyltributylammoniummethylsulfat eingesetzt wird.

13. Verbindungen der Formel (CDC) 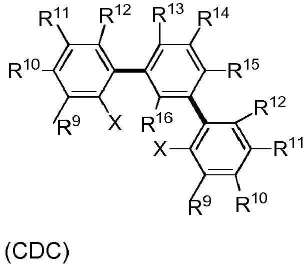
X = -OH oder -NR'R"
mit R' oder R" unabhängig voneinander ausgewählt aus der Gruppe umfassend Wasserstoff, *tert*-Butyloxycarbonyl, Methyloxycarbonyl, Benzyloxycarbonyl, Phenyloxycarbonyl, Acetyl, Trifluoracetyl, Benzoyl, Sulfonyl und Sulfenyl, vorzugsweise bestehend aus Wasserstoff, *tert*-Butyloxycarbonyl, Methyloxycarbonyl, Benzyloxycarbonyl, Phenyloxycarbonyl, Acetyl, Trifluoracetyl und Benzoyl, bevorzugt bestehend aus Wasserstoff, Acetyl und Trifluoracetyl; und besonders bevorzugt sind R' und R" Wasserstoff, oder R' und R" sind miteinander kovalent verknüpft, vorzugsweise über eine der nachfolgenden Gruppen PGa oder PGb 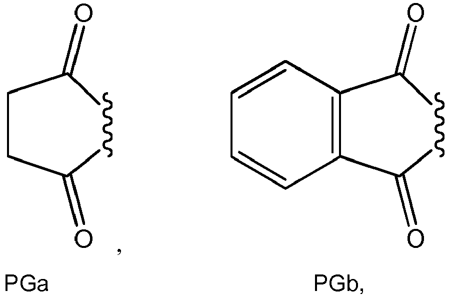
und R⁹, R¹⁰, R¹¹ und R¹² unabhängig voneinander ausgewählt sind aus der Gruppe enthaltend Wasserstoff, (C₁-C₁₂)-Alkyl, O-(C₁-C₁₂)-Alkyl, -N[(C₁-C₁₂)-Alkyl]₂, - NH(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, O-(C₁-C₁₂)-Alkyl, -N[(C₁-C₁₂)-Alkyl]₂ und Halogen, bevorzugt bestehend aus Wasserstoff, (C₁-C₁₂)-Alkyl, O-(C₁-C₁₂)-Alkyl und -N[(C₁-C₁₂)-Alkyl]₂;
und R¹³ und R¹⁵ unabhängig voneinander ausgewählt sind aus der Gruppe enthaltend (C₁-C₁₂)-Alkyl, O-(C₁-C₁₂)-Alkyl, -N[(C₁-C₁₂)-Alkyl]₂;
und R¹⁴ und R¹⁶ Wasserstoff sind,
wobei benachbarte Reste aus der Gruppe der Reste R⁹ bis R¹² über eine kovalente Bindung verbunden sein können.

14. Verbindungen gemäß Anspruch 13,
**dadurch gekennzeichnet,**
**dass** sie den Formeln 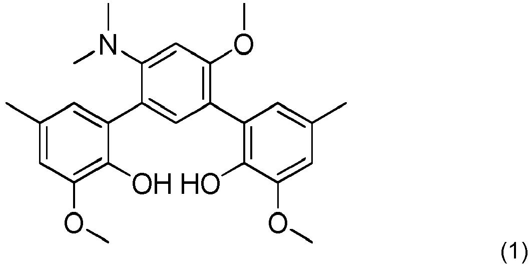 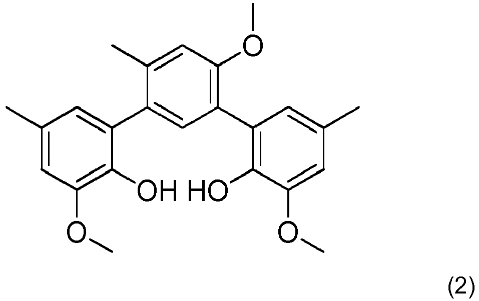 genügen.

15. Verwendung einer Verbindung gemäß Anspruch 13 oder 14 als Ligand zur Herstellung von Metall-Ligand-Katalysatorsystemen.

## Claims

1. Process for preparing compounds of the formula (ABA) 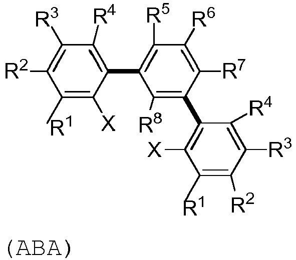 by reacting a compound of the formula (A) 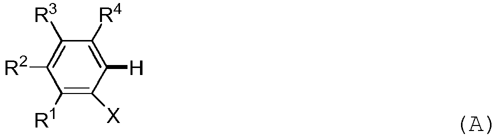 with a compound of the formula (B) 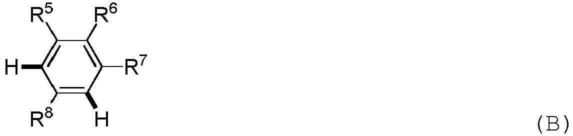
with X = -OH or -NR'R";
and R' = H or a protecting group function, preferably selected from the group comprising *tert*-butyloxycarbonyl, methyloxycarbonyl, benzyloxycarbonyl, phenyloxycarbonyl, acetyl, trifluoroacetyl, benzoyl, sulphonyl and sulphenyl; and R" = H or a protecting group function, preferably selected from the group comprising *tert*-butyloxycarbonyl, methyloxycarbonyl, benzyloxycarbonyl, phenyloxycarbonyl, acetyl, trifluoroacetyl, benzoyl, sulphonyl and sulphenyl; or R' and R" may together form covalently bonded protecting group radicals, preferably radicals of the formula PGa or PGb 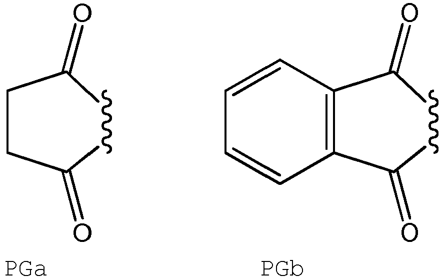
and R¹, R², R³, R⁴, R⁶ and R⁸ are each independently selected from the group comprising hydrogen, (C₁-C₁₂)-alkyl, -O-(C₁-C₁₂)-alkyl, -N[(C₁-C₁₂)-alkyl]₂, -NH(C₁-C₁₂)-alkyl, (C₆-C₂₀)-aryl, O-(C₆-C₂₀)-aryl and halogen, preferably consisting of hydrogen, (C₁-C₁₂)-alkyl, -O-(C₁-C₁₂)-alkyl, -N[(C₁-C₁₂)-alkyl]₂ and halogen, more preferably consisting of hydrogen, (C₁-C₁₂)-alkyl and -O-(C₁-C₁₂)-alkyl;
and R⁵ and R⁷ are each independently selected from the group comprising (C₁-C₁₂)-alkyl, -O-(C₁-C₁₂)-alkyl, -N[(C₁-C₁₂)-alkyl]₂, -NH(C₁-C₁₂)-alkyl, (C₆-C₂₀)-aryl, -0- (C₆-C₂₀)-aryl, halogen, preferably consisting of (C₁-C₁₂)-alkyl, -O-(C₁-C₁₂)-alkyl, -N[(C₁-C₁₂)-alkyl]₂ and halogen, and more preferably consisting of (C₁-C₁₂)-alkyl, -O-(C₁-C₁₂)-alkyl and -N[(C₁-C₁₂)-alkyl]₂;
where adjacent radicals from the group of the R¹ to R⁸ radicals may be joined via a covalent bond, **characterized in that**
the reaction is conducted electrochemically.

2. Process according to Claim 1,
**characterized in that**
the electrochemical process step is conducted in such a way that
aa) a mixture of at least one solvent and at least one conductive salt is produced,
bb) the compounds to be converted are added to this mixture, with addition of the compound of the formula (A) in a molar excess based on the compound (B),
cc) at least two electrodes are introduced into the reaction solution obtained in bb) and a voltage is applied to the electrodes,
dd) the compounds (A) and (B) are converted to the compound (ABA),
ee) the voltage is switched off, and optionally ff) the compound (ABA) is isolated and/or purified.

3. Process according to either of Claims 1 and 2, **characterized in that**
the molar ratio of the compound (A) to the compound (B) is in the range from 1.5:1 to 4:1, preferably from 1.8:1 to 2.5:1 and more preferably 2:1.

4. Process according to any of Claims 1 to 3, **characterized in that**
compounds of the formula (A) used are compounds in which X = NHR'.

5. Process according to any of Claims 1 to 3, **characterized in that**
compounds of the formula (A) used are compounds in which X = -NH₂.

6. Process according to any of Claims 1 to 5, **characterized in that**
R⁶ and R⁸ are identical and are preferably hydrogen.

7. Process according to any of Claims 1 to 6, **characterized in that**
the reaction is conducted in the presence of a solvent and the solvent used is a solvent from the group of acetonitrile, propylene carbonate, methyl carbonate, nitromethane, ethylene glycol dimethyl ether, methanesulphonic acid, benzene, toluene, water, methanol, ethanol, propanol, isopropanol, halogenated solvents or halogenated or non-halogenated acids or mixtures thereof.

8. Process according to Claim 6,
**characterized in that**
solvents used are methanol, formic acid, trifluoroacetic acid, hexafluoroisopropanol or mixtures thereof, preferably methanol, hexafluoroisopropanol or mixtures thereof, and preferably hexafluoroisopropanol.

9. Process according to any of Claims 1 to 8, **characterized in that**
the reaction is conducted without the use of organic oxidizing agents.

10. Process according to any of Claims 1 to 9, **characterized in that**
the electrochemical process step is conducted in the presence of at least one conductive salt, the conductive salts used preferably being those selected from the group of tetra(C₁-C₆-alkyl)ammonium and 1,3-di(C₁-C₆-alkyl)imidazolium salts, with the proviso that the alkyl groups may be halogen-substituted.

11. Process according to Claim 9,
**characterized in that**
the counterions of the conductive salts are selected from the group comprising arsenate, sulphate, hydrogensulphate, alkylsulphate, alkylphosphate, perchlorate, fluoride, arylsulphate, hexafluorophosphate and tetrafluoroborate.

12. Process according to Claim 9 or 10, **characterized in that**
the conductive salt used is a salt from the group comprising quaternary ammonium borates, ammonium fluoroalkylphosphates, ammonium fluoroalkylarsenates, ammonium trifluoromethylsulphonates, ammonium bis(fluoromethanesulphon)imides, ammonium tris(fluoromethanesulphonyl)methides, methyltributylammonium methylsulphate, methyltriethylammonium methylsulphate, tetrabutylammonium hexafluorophosphate, tetraethylammonium tetrafluoroborate, lithium hexafluorophosphate or tetraethylammonium tetrafluoroborate, preferably methyltriethylammonium methylsulphate or Bu3NMe+MeOSO3- (methyltributylammonium methylsulphate) and more preferably methyltributylammonium methylsulphate.

13. Compounds of the formula (CDC) 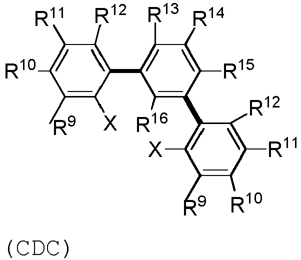
X = -OH or -NR'R";
with R' or R" each independently selected from the group comprising hydrogen, *tert-*butyloxycarbonyl, methyloxycarbonyl, benzyloxycarbonyl, phenyloxycarbonyl, acetyl, trifluoroacetyl, benzoyl, sulphonyl and sulphenyl, preferably consisting of hydrogen, *tert-*butyloxycarbonyl, methyloxycarbonyl, benzyloxycarbonyl, phenyloxycarbonyl, acetyl, trifluoroacetyl and benzoyl, preferably consisting of hydrogen, acetyl and trifluoroacetyl;
and, more preferably, R' and R" are hydrogen, or R' and R" are joined covalently to one another, preferably via one of the following groups PGa or PGb: 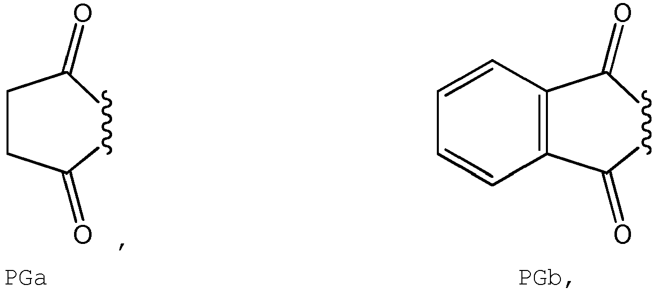
and R⁹, R¹⁰, R¹¹ and R¹² are each independently selected from the group comprising hydrogen, (C₁-C₁₂)-alkyl, O-(C₁-C₁₂)-alkyl, -N[(C₁-C₁₂)-alkyl]₂, -NH (C₁-C₁₂)-alkyl, (C₆-C₂₀)-aryl, -0- (C₆-C₂₀)-aryl and halogen, preferably consisting of hydrogen, (C₁-C₁₂)-alkyl, O-(C₁-C₁₂)-alkyl, -N[(C₁-C₁₂)-alkyl]₂ and halogen, more preferably consisting of hydrogen, (C₁-C₁₂)-alkyl, -O-(C₁-C₁₂)-alkyl and -N[(C₁-C₁₂)-alkyl]₂;
and R¹³ and R¹⁵ are each independently selected from the group comprising (C₁-C₁₂)-alkyl, O-(C₁-C₁₂)-alkyl, -N[(C₁-C₁₂)-alkyl]₂;
and R¹⁴ and R¹⁶ are hydrogen,
where adjacent radicals from the group of the R⁹ to R¹² radicals may be joined via a covalent bond.

14. Compounds according to Claim 13, **characterized in that**
they satisfy the formulae

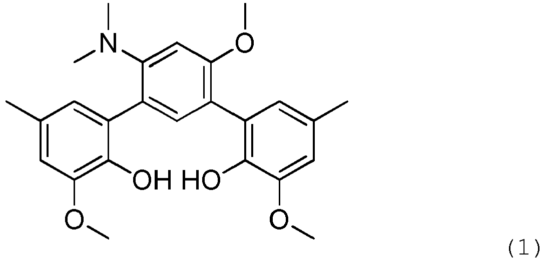

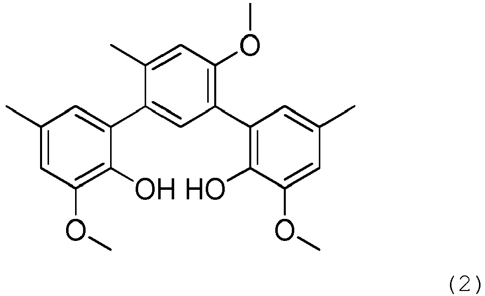

15. Use of a compound according to Claim 13 or 14 as ligand for preparation of metal-ligand catalyst systems.

## Revendications

1. Procédé de fabrication de composés de formule (ABA) 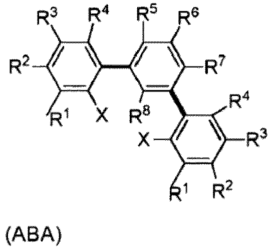 par mise en réaction d'un composé de formule (A) 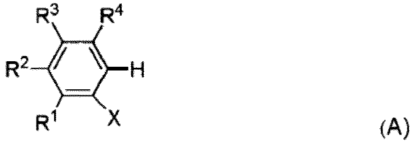 avec un composé de formule (B) 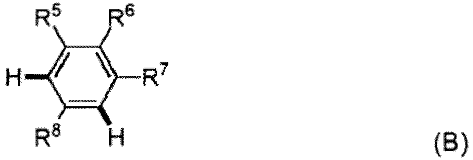
avec X = -OH ou -NR'R" ;
et R' = H ou une fonction de groupe protecteur, de préférence choisie dans le groupe comprenant *tert-*butyloxycarbonyle, méthyloxycarbonyle, benzyloxycarbonyle, phényloxycarbonyle, acétyle, trifluoroacétyle, benzoyle, sulfonyle et sulfényle ;
et R" = H ou une fonction de groupe protecteur, de préférence choisie dans le groupe comprenant *tert-*butyloxycarbonyle, méthyloxycarbonyle, benzyloxycarbonyle, phényloxycarbonyle, acétyle, trifluoroacétyle, benzoyle, sulfonyle et sulfényle ;
ou R' et R" étant des radicaux de groupes protecteurs reliés l'un à l'autre de manière covalente, de préférence des radicaux de formule PGa ou PGb 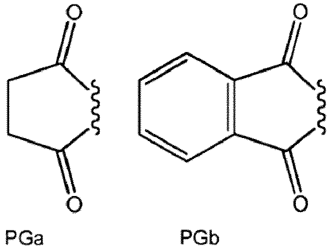
et R¹, R², R³, R⁴, R⁶ et R⁸ étant choisis indépendamment les uns des autres dans le groupe contenant hydrogène alkyle en (C₁-C₁₂), -O-alkyle en (C₁-C₁₂), -N[alkyle en (C₁-C₁₂)]₂, -NH-alkyle en (C₁-C₁₂), aryle en (C₆-C₂₀), 0-aryle en (C₆-C₂₀)et halogène, de préférence constitué par hydrogène, alkyle en (C₁-C₁₂), -O-alkyle en (C₁-C₁₂), - N [alkyle en (C₁-C₁₂)]₂ et halogène, avantageusement constitué par hydrogène, alkyle en (C₁-C₁₂)et -O-alkyle en (C₁-C₁₂) ;
et R⁵ et R⁷ étant choisis indépendamment l'un de l'autre dans le groupe contenant alkyle en (C₁-C₁₂), -O-alkyle en (C₁-C₁₂), -N[alkyle en (C₁-C₁₂)]₂, -NH-alkyle en (C₁-C₁₂), aryle en (C₆-C₂₀), O-aryle en (C₆-C₂₀) et halogène, de préférence constitué par alkyle en (C₁-C₁₂), -O-alkyle en (C₁-C₁₂), -N [alkyle en (C₁-C₁₂)]₂ et halogène, et avantageusement constitué par alkyle en (C₁-C₁₂), -0-alkyle en (C₁-C₁₂) et -N [alkyle en (C₁-C₁₂)]₂ ;
des radicaux voisins du groupe des radicaux R¹ à R⁸ pouvant être reliés par une liaison covalente, **caractérisé en ce que** la réaction est réalisée électrochimiquement.

2. Procédé selon la revendication 1, **caractérisé en ce que** l'étape de procédé électrochimique est réalisée de la manière suivante :
aa) un mélange d'au moins un solvant et d'au moins un sel conducteur est généré,
bb) les composés à mettre en réaction sont ajoutés à ce mélange, le composé de formule (A) étant ajouté en excès molaire par rapport au composé (B),
cc) au moins deux électrodes sont introduites dans la solution réactionnelle obtenue en bb) et une tension est appliquée aux électrodes,
dd) les composés (A) et (B) sont mis en réaction pour former le composé (ABA),
ee) la tension est arrêtée, et éventuellement ff) le composé (ABA) est isolé et/ou purifié.

3. Procédé selon l'une quelconque des revendications 1 ou 2, **caractérisé en ce que** le rapport molaire entre le composé (A) et le composé (B) se situe dans la plage allant de 1,5:1 à 4:1, de préférence de 1,8:1 à 2,5:1 et est de manière particulièrement préférée de 2:1.

4. Procédé selon l'une quelconque des revendications 1 à 3, **caractérisé en ce que** des composés dans lesquels X = -NHR' sont utilisés en tant que composé de formule (A).

5. Procédé selon l'une quelconque des revendications 1 à 3, **caractérisé en ce que** des composés dans lesquels X = -NH₂ sont utilisés en tant que composé de formule (A).

6. Procédé selon l'une quelconque des revendications 1 à 5, **caractérisé en ce que** R⁶ et R⁸ sont identiques et signifient de préférence hydrogène.

7. Procédé selon l'une quelconque des revendications 1 à 6, **caractérisé en ce que** la réaction est réalisée en présence d'un solvant et un solvant du groupe constitué par l'acétonitrile, le carbonate de propylène, le carbonate de méthyle, le nitrométhane, l'éther diméthylique d'éthylène glycol, l'acide méthanesulfonique, le benzène, le toluène, l'eau, le méthanol, l'éthanol, le propanol, l'isopropanol, les solvants halogénés ou les acides halogénés ou non halogénés ou les mélanges de ceux-ci est utilisé en tant que solvant.

8. Procédé selon la revendication 6, **caractérisé en ce que** du méthanol, de l'acide formique, de l'acide trifluoroacétique, de l'hexafluoroisopropanol ou des mélanges de ceux-ci, de préférence du méthanol, de l'hexafluoroisopropanol ou des mélanges de ceux-ci, et avantageusement de l'hexafluoroisopropanol, sont utilisés en tant que solvant.

9. Procédé selon l'une quelconque des revendications 1 à 8, **caractérisé en ce que** la réaction est réalisée sans utiliser d'oxydants organiques.

10. Procédé selon l'une quelconque des revendications 1 à 9, **caractérisé en ce que** l'étape de procédé électrochimique est réalisée en présence d'au moins un sel conducteur, des sels conducteurs choisis dans le groupe constitué par les sels de tétra(alkyle en C₁-C₆)-ammonium ou de (1,3-di(alkyle en C₁-C₆)imidazolium étant de préférence utilisés en tant que sels conducteurs, à condition que les groupes alkyle puissent être substitués par des halogènes.

11. Procédé selon la revendication 9, **caractérisé en ce que** les contre-ions des sels conducteurs sont choisis dans le groupe comprenant l'arsenate, le sulfate, l'hydrogénosulfate, l'alkylsulfate, l'alkylphosphate, le perchlorate, le fluorure, l'arylsulfate, l'hexafluorophosphate et le tétrafluoroborate.

12. Procédé selon la revendication 9 ou 10, **caractérisé en ce qu'**un sel du groupe comprenant les borates d'ammonium quaternaire, les fluoroalkylphosphates d'ammonium, les fluoroalkylarsenates d'ammonium, les trifluorométhylsulfonates d'ammonium, les bis(fluorométhanesulfone)imides d'ammonium, les tris-(fluorométhanesulfonyl)méthides d'ammonium, le méthylsulfate de méthyltributylammonium, le méthylsulfate de méthyltriéthylammonium, l'hexafluorophosphate de tétrabutylammonium, le tétrafluoroborate de tétraéthylammonium, l'hexafluorophosphate de lithium ou le tétrafluoroborate de tétraéthylammonium, de préférence le méthylsulfate de méthyltriéthylammonium ou Bu₃NMe⁺MeOSO₃⁻ (méthylsulfate de méthyltributylammonium) et de manière particulièrement préférée le méthylsulfate de méthyltributylammonium, est utilisé en tant que sel conducteur.

13. Composés de formule (CDC) 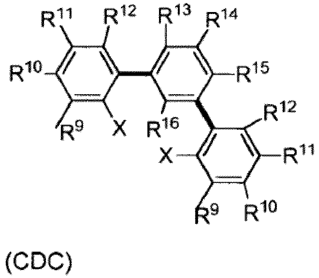
avec X = -OH ou -NR'R" ;
R' et R" étant choisis indépendamment l'un de l'autre dans le groupe comprenant hydrogène, *tert-*butyloxycarbonyle, méthyloxycarbonyle, benzyloxycarbonyle, phényloxycarbonyle, acétyle, trifluoroacétyle, benzoyle, sulfonyle et sulfényle ; de préférence constitué par hydrogène, *tert-*butyloxycarbonyle, méthyloxycarbonyle, benzyloxycarbonyle, phényloxycarbonyle, acétyle, trifluoroacétyle et benzoyle, avantageusement constitué par hydrogène, acétyle et trifluoroacétyle ;
et de manière particulièrement préférée, R' et R" représentant l'hydrogène, ou R' et R" étant reliés l'un à l'autre de manière covalente, de préférence par un des groupes PGa et PGb suivants : 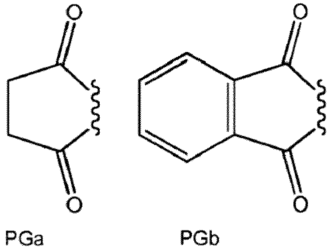
et R⁹, R¹⁰, R¹¹ et R¹² étant choisis indépendamment les uns des autres dans le groupe contenant hydrogène, alkyle en (C₁-C₁₂), -O-alkyle en (C₁-C₁₂), -N [alkyle en (C₁-C₁₂)]₂, - NH-alkyle en (C₁-C₁₂), aryle en (C₆-C₂₀), O-aryle en (C₆-C₂₀) et halogène, de préférence constitué par hydrogène, alkyle en (C₁-C₁₂), -O-alkyle en (C₁-C₁₂), -N [alkyle en (C₁-C₁₂)]₂ et halogène, avantageusement constitué par hydrogène, alkyle en (C₁-C₁₂), -O-alkyle en (C₁-C₁₂)et - N [alkyle en (C₁-C₁₂)]₂ ;
et R¹³ et R¹⁵ étant choisis indépendamment l'un de l'autre dans le groupe contenant alkyle en (C₁-C₁₂), -O-alkyle en (C₁-C₁₂), -N [alkyle en (C₁-C₁₂)]₂ ;
et R¹⁴ et R¹⁶ représentant l'hydrogène,
des radicaux voisins du groupe des radicaux R⁹ à R¹² pouvant être reliés par une liaison covalente.

14. Composés selon la revendication 13, **caractérisés en ce qu'**ils satisfont les formules

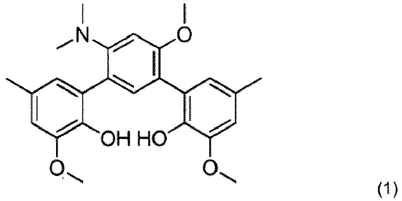

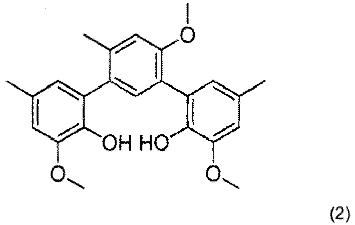

15. Utilisation d'un composé selon la revendication 13 ou 14 en tant que ligand pour la fabrication de systèmes catalytiques métal-ligand.
